# EUROPEAN PATENT APPLICATION

(11) **EP 2 272 519 A1**
(43) Date of publication of application: **12.01.2011**
(21) Application number: 08741598.0
(22) Date of filing: 14.04.2008
(51) Int. Cl.: A61K 31/57, A61K 9/16, A61P 15/12, A61P 15/06

(54) **METHOD AND PHARMACEUTICAL COMPOSITION FOR OBTAINING THE PLASMATIC PROGESTERONE LEVELS REQUIRED FOR DIFFERENT THERAPEUTIC INDICATIONS**

(71) Applicant: Posi Visionary Solutions LLP, Bromley, Kent BR2 9LZ (GB)
(72) Inventor: SAVOIR VILBOEUF, John Claude, C.P. 03100, México D.F. (MX); DE GYVES LÓPEZ LENA, Aurelio, C.P. 03100, México D.F. (MX); MARTÍNEZ DE LEÓN, Juan Ramón, C.P. 03100, México D.F. (MX)
(74) Representative: Maggs, Michael Norman
(86) International application number: PCT/MX2008/000051
(87) International publication number: WO 2009/128692

(57) **Abstract**

The invention relates to the development of a method and pharmaceutical compositions for obtaining plasmatic progesterone levels in humans and for maintaining a plasmatic progesterone concentration between 42 and 3.5 ng/mL for eight days as well as maximum plasmatic concentrations (Cmax) between 12 and 42 ng/mL, sufficient for use in different therapeutic options that require said progesterone concentrations.

## Description

### FIELD OF INVENTION

Present invention refers to a design of a method and pharmaceutical compositions for achieving and keeping progesterone plasma levels in humans between 42 and 3.5 ng/mL along 8 days as well as maximum plasma concentrations (Cmax) between 12 and 42 ng/mL, sufficient for application in several therapeutic conditions requiring said progesterone concentrations.

### TECHNICAL FIELD

### Background of Invention

There are a number of pharmaceutical compositions in the art for delivering drugs in controlled form and more particularly for providing hormones; however, in every case there are some significant drawbacks. For instance, it is reported that when progesterone is orally administered presents a drawback of suffering a wide liver metabolism due to an effect of first step; wherein formed metabolites may have secondary effects, in addition to a limited progesterone bioavailability through this way. Thus, orally administered therapies demand higher progesterone doses and therefore, they have a higher probability of adverse events due to a higher exposure.

The present invention constitutes an alternative for achieving more accurate plasma concentrations, reproducible and with a lower variation along a treatment from a smaller number and frequency of injections, facts which may not be achieved with therapies and progesterone containing products currently available. The method subject of present invention may be applicable within medical practice for several therapeutic conditions requiring progesterone such as: treatment of secondary amenorrhea, dysfunctional uterine hemorrhage, premenstrual syndrome when higher progesterone concentrations are required, progesterone replacement in ovariectomized women, progesterone complement in luteal phase support in assisted reproduction procedures, threatened abortion and relapsing abortion prevention by luteal insufficiency, premature labor, endometriosis, endometrial hyperplasia, hirsutism, and others.

The method of present invention allows a longer permanence of progesterone in plasma within required therapeutic levels for conditions described in above paragraph for up to 8 days, without the risk observed with conventional therapies and preventing that a repeated progesterone administration may lead to variations in plasma concentrations observed in therapies known up to date.

The present invention is applicable to progesterone administered as an injectable suspension, allowing obtaention of required plasma concentration ranges demanding progesterone in page 2. This is also applicable to any type of injectable suspension regardless of the geometric shape of active principle particles, that is, applicable to particle suspensions with a well-defined geometric shape such as spherical microparticles or as crystals without a defined geometric shape. In the light of above, the closest state of the art for this invention are US patents 5,360,616 ('616) and U.S. 5,643,604 ('604), both in the name of Aplicaciones Farmacéuticas, S. A. de CV.

Patent ('616) refers to injectable pharmaceutical compositions of modified release medicinal products consisting of non-porous solid steroid microspheres from 1 to 300 microns, manufactured by a spray and freezing process. However, this patent only refers to a modified release microsphere formulation and its manufacturing process but does not disclose possible applications of in-vivo modified release which allows reaching plasma levels in humans useful for several therapeutic options with progesterone, particularly those disclosed in page 2.

Patent ('604) related to a parenteral dosage form wherein the active principle is comprised in microspheres that is, in spherical shape structures determined by one or more pharmacologically inactive carrier substance, but not disclosing possible applications of its in-vivo modified release which allows reaching plasma levels in humans useful for several therapeutic options mainly with progesterone for luteal phase support.

Known medical compositions up to now have not achieved to maintain during more than one day with a single dose suitable progesterone plasma concentrations for therapies demanding progesterone, particularly those described in page 2.

### SUMMARY OF INVENTION

However, the present invention includes a method for achieving and maintaining more accurate, reproducible plasma concentrations and with less variation along referred treatments, facts which are not achieved with pharmaceutical therapies and products available up to date.

In another embodiment from the present invention, progesterone is possible to be administered in a single injection or multiple injections since from the first injection progesterone permanence in plasma is possible to be reached within the required range for the conditions described in page 2.

Still in another embodiment of the invention a pharmaceutical composition is provided in the form of an injectable suspension of spherical-shape microparticles or microparticles without any defined geometric shape.

### OBJECTIVES OF THE INVENTION

It is an object of present invention to provide a therapeutic option for progesterone weekly administration to prevent daily administration, even two or three times a day in order to maintain progesterone plasma levels along 8 days between 13 and 3.5 ng/mL with a single 100 mg progesterone injection.

Another object of present invention in an alternative embodiment is to keep progesterone plasma levels during 8 days between 20 and 7 ng/mL with a 200 mg progesterone single injection.

Another object of present invention in an alternative embodiment is to maintain progesterone plasma levels between 26 and 8 ng/mL with four weekly injections of 200 mg progesterone.

Another object of the invention is in an alternative embodiment to maintain progesterone plasma levels between 42 and 13 ng/mL with four weekly injections of 300 mg progesterone.

Another object of the invention is to decrease the administration frequency and amount of orally required progesterone in order to decrease the possibility of adverse events caused by a frequent and prolonged exposure to progesterone.

Another object of the invention is to prevent maximum peaks of plasma concentration observed after administering progesterone oily solutions and possible adverse events associated with high plasma concentrations of progesterone caused by said maximum peaks.

Another object of present invention is to decrease traumatic events and local irritability observed with injectable oily solutions comprising progesterone, requiring a daily injection.

Another object of the invention is to prevent upset and dose inconsistency observed with intravaginal administration, the lack of reproducibility of the absorbed amount of progesterone and the fact that plasma concentrations rapidly decrease such that therapeutic levels are not possible to be maintained more than 24 hours.

Still another object of invention is to provide progesterone according to the invention, in a single-injection or repeated-injection scheme.

Still another object of the invention is to provide progesterone in the form of microspheres or microcrystals in an injectable suspension.

### BRIEF DESCRIPTION OF THE DRAWINGS

Figure 1 is a graph of time-dependant progesterone plasma concentration after a single injection of a 100 mg injectable suspension of progesterone microspheres.
Figure 2 is a graph of time-dependant progesterone plasma concentration after a single injection of a 200 mg injectable suspension of progesterone microspheres.
Figure 3 is a graph of time-dependant progesterone plasma concentration after four injections of a 200 mg injectable suspension of progesterone microspheres.
Figure 4 is a graph of time-dependant progesterone plasma concentration after four injections of a 300 mg injectable suspension of progesterone microspheres.
Figure 5 is a graph of time-dependant progesterone plasma concentration depending on time, after four injections of a 300 mg injectable suspension of progesterone microcrystalline particles not having a defined geometric shape.

### DETAILED DESCRIPTION OF INVENTION

The present invention, provides a method and pharmaceutical compositions preferably for weekly administration, wherein progesterone plasma concentration in required therapeutic levels is maintained for certain treatments with this hormone.

Progesterone plasma levels along 8 days are between 13 and 3.5 ng/mL with a 100 mg single progesterone injection, between 20 and 7 ng/mL with a 200 mg single progesterone injection, between 26 and 8 ng/mL after four 200 mg progesterone injections and between 42 and 13 ng/mL after four 300 mg progesterone injections, all those in the form of an injectable suspension of progesterone particles.

Progesterone release in human body is such that favors its permanence in the required concentration ranges for several therapeutic options demanding progesterone. In an ideal situation, progesterone containing drug dissolution is directly proportional to microsphere or microcrystalline particle erosion speed on injection site and does not depend on geometry thereof.

### EXAMPLES

Following examples illustrate some preferred embodiments of the invention wherein plasma levels are accurately achieved during 8 days

### Example 1

SINGLE 100 mg INJECTION OF PROGESTERONE SPHERICAL MICROPARTICLES IN AN INJECTABLE SUSPENSION.

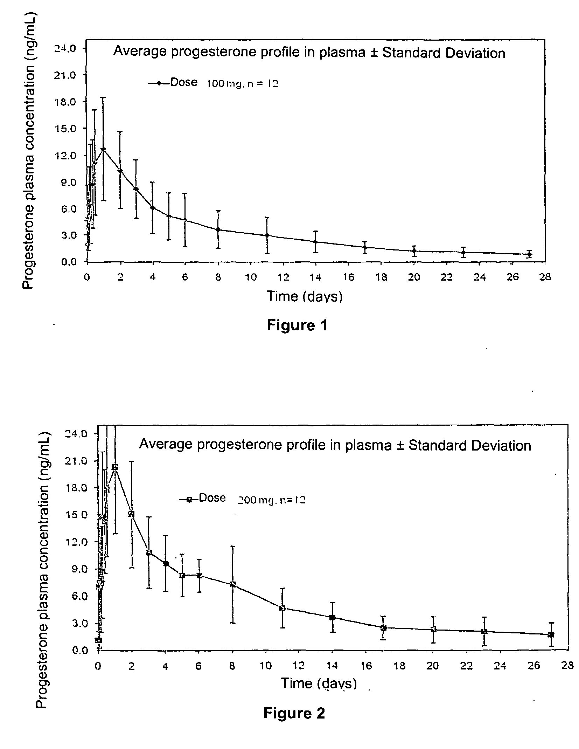

12 post-menopausal women were administered with a 100 mg single injection of progesterone spherical microparticles, in the form of an injectable aqueous suspension. Obtained plasma levels are illustrated in Figure 1, wherein progesterone plasma concentrations are observed to be maintained up to 7 days in suitable levels for several therapies requiring said progesterone concentrations.

### Example 2

SINGLE 200 mg INJECTION OF PROGESTERONE SPHERICAL MICROPARTICLES IN AN INJECTABLE SUSPENSION.

12 post-menopausal women were administered with a single 200 mg injection of progesterone spherical microparticles in the form of an injectable aqueous suspension. Obtained plasma levels are illustrated in Figure 2, wherein progesterone plasma concentrations are observed to be maintained up to 7 days in suitable levels for several therapies requiring said progesterone concentrations and described in page 2.

### Example 3

### REPEATED 200 mg INJECTIONS OF PROGESTERONE SPHERICAL MICROPARTICLES IN AN INJECTABLE SUSPENSION.

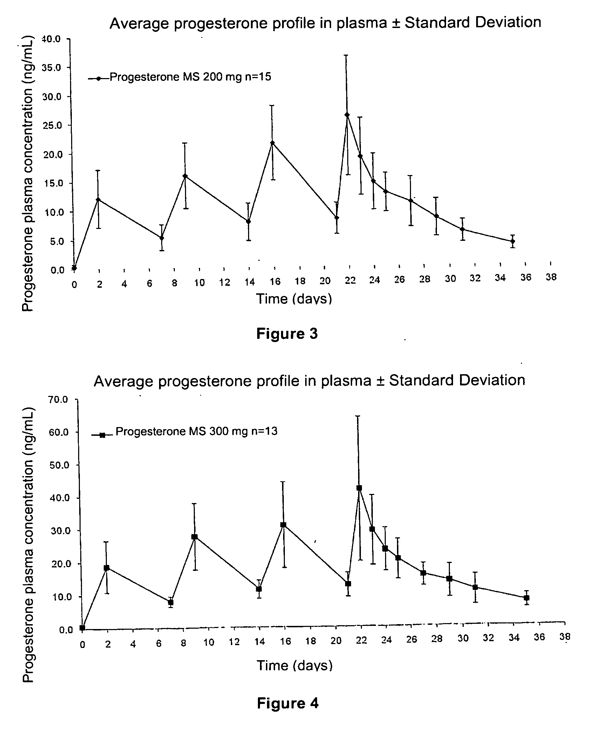

Four 200 mg repeated injections of progesterone spherical microparticles were administered in the form of an injectable aqueous suspension to 15 post-menopausal women. Obtained plasma levels are illustrated in Figure 3, wherein progesterone plasma concentrations are observed to be maintained up to 7 days in suitable levels for several therapies requiring said progesterone concentrations and described in page 2.

### Example 4

### REPEATED 300 mg INJECTIONS OF INJECTION OF PROGESTERONE SPHERICAL MICROPARTICLES IN AN INJECTABLE SUSPENSION.

Four 300 mg repeated injections of progesterone spherical microparticles were administered in the form of an injectable aqueous suspension to 13 post-menopausal women. Obtained plasma levels are illustrated in Figure 4, wherein progesterone plasma concentrations are observed to be maintained up to 7 days in suitable levels for several therapies requiring said progesterone concentrations and described in page 2.

### Example 5

### REPEATED 300 mg INJECTIONS OF CRYSTAL PROGESTERONE MICROPARTICLES WITHOUT DEFINED SHAPE IN AN INJECTABLE SUSPENSION.

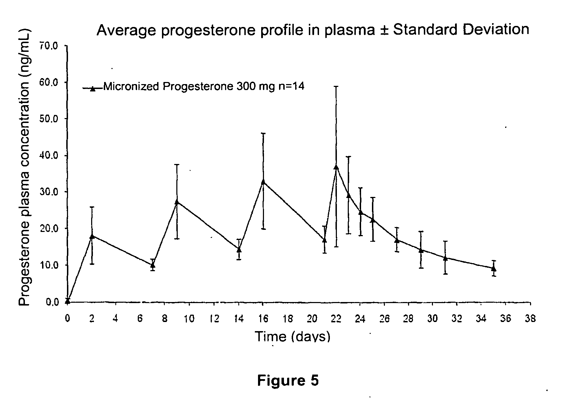

14 post-menopausal women were administered with 4 repeated injections of 300 mg progesterone microparticles without a defined geometric shape, in an injectable aqueous suspension. Obtained plasma levels are illustrated in Figure 5, wherein progesterone plasma concentrations are observed to be maintained up to 7 days in suitable levels for several therapies requiring said progesterone concentrations and described in page 2.

## Claims

1. A method for maintaining sustained plasma progesterone concentrations in humans between 42 and 3.5 ng/mL during 8 days and maximum plasma concentrations (Cmax) between 12 and 42 ng/mL sufficient for application in different therapeutic options requiring said progesterone concentrations.

2. The method according to claim 1, being sufficient to maintain progesterone plasma concentrations between 13 and 3.5 ng/mL from administration of a single 100 mg progesterone injection in the form of an injectable suspension.

3. The method according to claim 2, wherein application is in secondary amenorrhea, dysfunctional uterine hemorrhage, premenstrual syndrome, progesterone replacement in ovariectomized women.

4. The method according to claim 1, wherein progesterone plasma concentrations are maintained between 20 and 7 ng/mL from administration of a single 200 mg progesterone injection in the form of an injectable suspension.

5. The method according to claim 1, wherein progesterone plasma concentrations are maintained between 26 and 8 ng/mL from 200 mg multiple progesterone injection in the form of injectable suspension.

6. The method according to claim 4, wherein application is luteal phase complement in assisted reproduction procedures, threatened abortion and prevention of relapsing abortion by luteal insufficiency, premature labor, endometriosis, endometrial hyperplasia and hirsutism, in secondary amenorrhea, dysfunctional uterine hemorrhage and premenstrual syndrome when higher progesterone levels are required.

7. The method according to claim 1, for maintaining progesterone plasma concentrations between 42 and 13 ng/mL from administering multiple 300 mg injections of progesterone in the form of an injectable suspension.

8. The method according to claim 7, wherein application is a luteal phase complement in assisted reproduction procedures, threatened abortion and prevention of relapsing abortion by luteal insufficiency, premature labor, endometriosis, endometrial hyperplasia and hirsutism, in secondary amenorrhea, dysfunctional uterine hemorrhage and premenstrual syndrome when higher levels of progesterone are required.

9. The method according to claim 1, obtained from an injectable aqueous suspension of progesterone particles.

10. The method according to claim 9, obtained from a single injection or sole dose from an injectable aqueous suspension of spherical shape progesterone particles.

11. The method according to claim 9, obtained from multiples injections or multiple doses of an injectable aqueous suspension of spherical shape progesterone particles.

12. The method according to claim 9, obtained from an injectable aqueous suspension of progesterone particles without a defined geometric shape.

13. The method according to claim 12, obtained from a single injection or sole dose of an injectable aqueous suspension of progesterone particles without a defined geometric shape.

14. The method according to claim 12, obtained from repeated injections of an injectable aqueous suspension of progesterone particles without a defined geometric shape.

15. The method according to claim 1, obtained from an injectable suspension.

16. Injectable pharmaceutical composition for single injection of spherical shape progesterone particles.

17. Pharmaceutical composition according to claim 16, wherein progesterone particle shape lacks of a defined geometric shape.

18. Pharmaceutical composition according to claim 16, wherein progesterone particles not having a defined geometric shape are crystals which may be injected in the shape of injectable aqueous suspension.

19. Use of a pharmaceutical composition according to claims 16 to 18, for treatment of secondary amenorrhea, dysfunctional uterine hemorrhage, premenstrual syndrome (when higher progesterone concentrations are required), progesterone replacement in ovariectomized women, luteal phase complement in assisted reproduction procedures, threatened abortion and relapsing abortion prevention by luteal insufficiency, premature labor, endometriosis, endometrial hyperplasia, hirsutism, and others.

20. Injectable aqueous suspension of spherical shape progesterone particles.

21. Injectable aqueous suspension of progesterone crystals without a defined geometric shape.
